(19) Europäisches Patentamt
European Patent Office
Office européen des brevets

(11) Veröffentlichungsnummer: **0 143 075**
**B1**

(12) # EUROPÄISCHE PATENTSCHRIFT

(45) Veröffentlichungstag der Patentschrift: **19.11.87**

(51) Int. Cl.[4]: **C 07 D 301/27**, C 07 D 303/36

(21) Anmeldenummer: **84810462.6**

(22) Anmeldetag: **24.09.84**

(54) **Verfahren zur Herstellung von N-Glycidylverbindungen.**

(30) Priorität: **29.09.83 GB 8326118**
**29.09.83 GB 8326119**

(43) Veröffentlichungstag der Anmeldung: **29.05.85 Patentblatt 85/22**

(45) Bekanntmachung des Hinweises auf die Patenterteilung: **19.11.87 Patentblatt 87/47**

(84) Benannte Vertragsstaaten: **AT BE CH DE FR GB IT LI NL SE**

(56) Entgegenhaltungen:
**EP - A - 0 076 584**
**US - A - 2 951 825**

**CHEMICAL ABSTRACTS, Band 91, Nr. 5, 30. Juli 1979, Seite 617, Nr. 39297f, Columbus, Ohio, US**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: ***Dobinson, Bryan, Dr.*, 8 Temple Close, Duxford Cambridge CB2 4PX (GB)**
Erfinder: **Thoseby, Michael Robert, Dr., 29, De Freville Avenue, Cambridge CB4 1HW (GB)**

**EP 0 143 075 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von N-Glycidylverbindungen, speziell ein Verfahren zur Herstellung von aromatischen N-Glycidylaminen, insbesondere solchen, bei denen Epoxidharze mit im Durchschnitt mehr als einer Glycidylgruppe pro Molekül entstehen, sowie die dadurch erhaltenen N-Glycidylamine.

Epoxidharze werden oft als Klebstoffe, Überzüge, Giesskörper, Isoliermaterialien sowie in verstärkten Verbundstoffen verwendet, und eine Vielzahl chemisch verschiedener Epoxidharze ist im Handel erhältlich. Solche Harze sind üblicherweise Glycidylether oder -ester, hergestellt aus Epichlorhydrin und einem Bisphenol oder einer Dicarbonsäure. Für Anwendungen, die gute Eigenschaften bei hoher Temperatur erfordern, wie z.B. in der Luftfahrtindustrie, werden oft Harze mit Glycidylgruppen, die an aromatische Aminostickstoffatome gebunden sind, bevorzugt. Solche Stoffe werden durch Umsetzung von aromatischen Aminen mit etwa 0,8-10 Äquivalenten Epichlorhydrin pro Aminowasserstoffatom hergestellt, gefolgt von konventioneller Dehydrochlorierung der Produkte unter Anwendung von Basen. Diese Reaktion kann ohne Katalysator durchgeführt werden, oder wie in der britischen Patentschrift Nr. 2 111 977 beschrieben, in Anwesenheit eines sauren Katalysators.

Trotz ihren guten Eigenschaften können die auf konventionelle Art hergestellten N-Glycidylamine in zwei Beziehungen verbessert werden. Erstens liegt der Epoxidgehalt der so erhaltenen Produkte selten in der Nähe der theoretischen Werte für vollständige Glycidylierung, d.h. der Werte, die man bestimmen würde, falls jedes Aminwasserstoffatom durch eine Glycidylgruppe ersetzt wäre. Der tatsächliche Epoxidgehalt variiert je nach Art des Amins, und ist insbesondere davon abhängig, ob andere Substituenten im Molekül vorhanden sind. So wird z.B. der Epoxidgehalt des handelsüblichen glycidylierten Bis-(aminophenyl)methans in der Kirk-Othmer «Encyclopedia of Chemical Technology», 3. Auflage, Band 9, Seite 277 mit 117-133 angegeben. Dies entspricht einem Epoxidgehalt von 79-90% des theoretisch möglichen Wertes. Es ist bekannt, dass die Eigenschaften des gehärteten Harzes vom Epoxidgehalt des nicht vernetzten Harzes abhängen: je höher der Epoxidgehalt, desto grösser die Vernetzungsdichte und folglich desto stärker das vernetzte Harz. Es ist eindeutig, dass ein höherer Epoxidgehalt des Harzes von Vorteil wäre.

Der zweite Nachteil von konventionell hergestellten N-Glycidylaminen ist, dass sie oft sehr viskos sind, wahrscheinlich als Folge einer Nebenreaktion während der Herstellung, in der eine Kupplungsreaktion statt der gewünschten Glycidylierung stattfindet. Solche Kupplungsreaktionen verursachen auch die erwähnten tieferen Epoxidgehalt-Werte. Die Verwendung viskoser Harze verursacht Schwierigkeiten, insbesondere bei der Herstellung von faserverstärkten Verbundstoffen oder Giesskörpern, wodurch oft die Anwendung von inerten Verdünnern, die die Viskosität reduzieren, oft nötig wird.

Im allgemeinen wird die Verwendung von Verdünnern als unerwünscht erachtet. Reaktive Verdünner sind solche, die mit dem Härter reagieren und im vernetzten Harz verbleiben. Sie können sich nachteilig auf die Eigenschaften des gehärteten Harzes auswirken. Inerte Verdünner werden vor dem Härten durch Verdampfung entfernt, und stellen oft eine Gefahr wegen ihrer Brennbarkeit oder Giftigkeit dar. Ausserdem können sie die Eigenschaften des gehärteten Harzes beeinträchtigen, falls sie nicht vollständig aus dem Harz entfernt werden.

Es wurde nun gefunden, dass Produkte mit höherem Epoxidgehalt und niedrigerer Viskosität erhalten werden, wenn die N-Glycidylierung von aromatischen Aminen in Anwesenheit von zwei- oder mehrwertigen Metallsalzen der Salpetersäure oder der Perchlorsäure, oder mit zwei- oder mehrwertigen Metallsalzen einer Halogen-enthaltenden Carbonsäure oder Sulfonsäure, die mit einem oder mehreren Fluor-, Chlor- oder Bromatomen am α-Kohlenstoffatom zur Carboxyl- oder Sulfonsäuregruppe substituiert ist, als Katalysator, durchgeführt wird. Ein weiterer überraschender Vorteil des erfindungsgemässen Verfahrens ist die Tatsache, dass aromatische Amine, deren Aminogruppe oder -gruppen durch andere Gruppen im Molekül sterisch gehindert sind, verhältnismässig leicht N-glycidyliert werden können, wodurch die Anzahl aromatischer Amine, aus denen Epoxidharze hergestellt werden können, wesentlich vergrössert wird.

Zur Illustrierung der Nachteile von Verfahren zur Herstellung von N-Glycidylaminen unter Verwendung von bekannten Katalysatoren kann erwähnt werden, dass gemäss der oben erwähnten GB Patentschrift Nr. 2 111 977 mit Trifluormethansulfonsäure als Katalysator N-Glycidylamine in relativ schlechter Ausbeute und mit tiefem Epoxidgehalt sowie mit hoher Viskosität erhalten werden.

Die Anwendung von Metallsalzen anorganischer Oxy-Säuren als Härtungsbeschleuniger bei der Härtung von Epoxidharzen mit Aminen ist bekannt. Die GB Patentschrift Nr. 1 464 045 beschreibt härtbare Stoffgemische enthaltend (a) ein Epoxidharz, (b) ein aromatisches oder cycloaliphatisches Polyamin und (c) Magnesium-, Calcium-, Zink-, Mangan-, Kobalt- oder Nickelperchlorat. Die GB Patentschrift Nr. 1 521 356 beschreibt härtbare Stoffgemische enthaltend (a) ein Epoxidharz, (b) eine aromatische Aminoverbindung und (c) ein Nitrat von Magnesium oder von einem zwei- oder mehrwertigen Metall der Gruppe IIb, IIIb, IVb, Vb, VIb, VIIb oder VIII des periodischen Systems.

Die Verwendung von Metallsalzen halogenierter Carbonsäuren und Sulfonsäuren als Härtungsbeschleuniger für die Härtung von Epoxidharzen mit Aminen ist ebenfalls bekannt. Die GB Patentschrift Nr. 1 498 542 beschreibt Stoffgemische, die als Härter für Epoxidharze geeignet sind, bestehend aus

a) u.a. einem Polyamin oder einem Polyaminoamid und

b) einer aliphatischen oder araliphatischen Monocarbonsäure mit 2 bis 8 Kohlenstoffatomen, die am Kohlenstoffatom in α-Stellung zur Carboxylgruppe mindestens zwei Halogenatome, ausgewählt aus Fluor und Chlor, enthält, bzw. Salzen dieser Säuren.

Die bevorzugten Metalle, deren Salze in den aromatische Amine enthaltenden Stoffgemischen verwendet werden, sind Lithium, Natrium Calcium und Magnesium.

Die GB Patentschrift Nr. 1 500 206 beschreibt Stoffgemische, die für die Verwendung als Härter für Epoxidharze geeignet sind, enthaltend (a) ein aromatisches, heterocyclisches oder cycloaliphatisches Polyamin, und (b) ein Salz der Trifluormethansulfonsäure. Bevorzugte Salze sind Salze von Lithium, Calcium, Zink, Cadmium, Kobalt, Nickel, Mangan und Magnesium.

Trotz deren Verwendung als Beschleuniger bei der Härtung von Epoxidharzen, war es bisher nicht bekannt, dass Metallsalze von anorganischen Oxy-Säuren oder von halogenierten Carbonsäuren oder Sulfonsäuren als Katalysatoren bei der Herstellung von N-Glycidylgruppen enthaltenden Epoxidharzen verwendet werden können, oder dass die auf diese Art hergestellten Harze überlegene Eigenschaften aufweisen.

Gegenstand der vorliegenden Erfindung ist deshalb ein Verfahren zur Herstellung von aromatischen N-Glycidylaminen, worin ein Amin, das mindestens ein und vorzugsweise mindestens zwei aromatische Aminwasserstoffatome enthält, mit mindestens 0,7 Äquivalenten und vorzugsweise mindestens 0,8 bis 1,5 Äquivalenten Epichlorhydrin pro Aminwasserstoffäquivalent des aromatischen Amins, in Anwesenheit eines zwei- oder mehrwertigen Metallsalzes (a) der Salpeter- oder der Perchlorsäure oder (b) einer Carbon- oder Sulfonsäure, die durch Fluor, Chlor oder Brom am α-Kohlenstoff zur Carboxyl- oder Sulfonsäuregruppe substituiert ist, erhitzt wird, und anschliessend das Produkt dehydrochloriert wird. Die vorliegende Erfindung betrifft auch aromatische N-Glycidylamine hergestellt nach dem vorliegenden Verfahren.

Die im erfindungsgemässen neuen Verfahren als Katalysatoren verwendeten Nitrate und Perchlorate sind vorzugsweise Salze von Metallen der Gruppen IIa, IIb, IIIb, VIIb oder VIII des periodischen Systems, wie im «Handbook of Chemistry», Lange, 12. Auflage, Mc-Graw-Hill veröffentlicht. Nitrate und Perchlorate des Magnesiums, Calciums, Zinks, Mangans, Nickels, Lanthans, Vanadiums (als Vanadyl), Ytterbiums und Urans (als Uranyl) sind besonders bevorzugt.

Bei den Salzen von Halogen-substituierten Carbonsäuren und Sulfonsäuren, die als Katalysatoren verwendet werden, sind die Anionen vorzugsweise von höchstens 4 Kohlenstoffatome enthaltenden Säuren abgeleitet. Besonders bevorzugt sind Salze von Trifluoressigsäure, Trifluormethansulfonsäure, Trichloressigsäure, 2,2-Dichlorpropionsäure und Tribromessigsäure. Die Kationen der Fluor-, Chlor- oder Brom-substituierten Carbon- oder Sulfonsäuren sind vorzugsweise solche der Metalle der Gruppe IIa und der Übergangsmetalle des periodischen Systems der Elemente wie im «Handbook of Chemistry», Lange, 12. Auflage, McGraw-Hill veröffentlicht. Besonders bevorzugt sind Kationen des Eisens, Zinks, Cadmiums und Lanthans, und ganz besonders bevorzugt sind Kationen des Magnesiums, Vanadiums (als Vanadyl), Mangans, Kobalts und Nickels.

Spezifisch bevorzugte Salze, die im erfindungsgemässen Verfahren als Katalysatoren verwendet werden, sind Magnesiumperchlorat, Calciumperchlorat, Zinkperchlorat, Nickelperchlorat, Magnesiumnitrat, Mangannitrat, Lanthannitrat, Ytterbiumnitrat, Uranylnitrat, Magnesiumtrifluoracetat, Mangantrifluoracetat, Nickeltrifluoracetat, Vanadyltrifluoracetat, Magnesiumtrifluormethansulfonat, Kobalttrifluormethansulfonat, Magnesiumtrichloracetat, Magnesium-2,2-dichlorpropionat und Magnesiumtribromacetat.

Die im Reaktionsgemisch verwendete Salzmenge beträgt im allgemeinen 0,1 bis 110 Gewichtsteile pro 100 Gewichtsteile des aromatischen Amins, insbesondere 0,4 bis 2 Gewichtsteile pro 100 Gewichtsteile des Amins.

Das aromatische Amin, das gemäss der vorliegenden Erfindung glycidyliert wird, kann nur primäre, nur sekundäre, oder primäre und sekundäre Aminogruppen, die direkt an einen aromatischen Ring gebunden sind, enthalten, und es kann einen oder mehrere aromatische Ringe enthalten. Diese aromatischen Ringe können auch durch weitere Gruppen substituiert sein, wie z.B. Alkylgruppen, insbesondere solche mit 1 bis 4 Kohlenstoffatomen, Alkylengruppen mit 1 bis 4 Kohlenstoffatomen, Sulfonylgruppen, Halogenatomen, Hydroxylgruppen, Alkoxygruppen mit 1 bis 4 Kohlenstoffatomen und tertiären Aminogruppen. Bevorzugt werden im vorliegenden Verfahren Amine mit einer oder zwei primären Aminogruppen verwendet. Insbesondere Aniline, Aminophenylindane und Amine der Formel I oder II

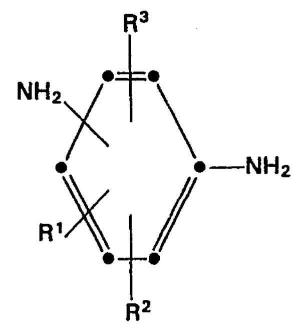

I

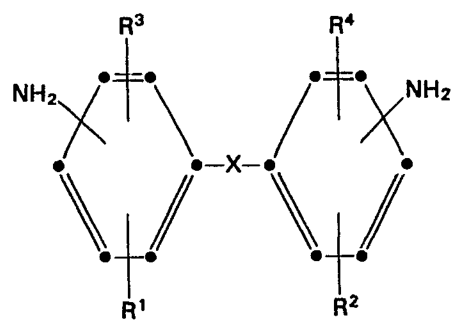

II

worin

$R^1$, $R^2$, $R^3$ und $R^4$ gleich oder verschieden sind und Alkylgruppen mit 1 bis 4 Kohlenstoffatomen oder Wasserstoffatome darstellen, und X eine direkte Bindung, eine Alkylengruppe mit 1 bis 4 Kohlenstoffatomen, ein Sauerstoffatom, ein Schwefelatom oder eine Carbonyl- oder Sulfonylgruppe darstellt, sind besonders bevorzugt.

Beispiele ganz besonders bevorzugter Amine sind Anilin, 1,3,3-Trimethyl-1-(4-aminophenyl)-5-aminoindan, 1,3,3-Trimethyl-1-(4-aminophenyl)-6-aminoindan, o-, m- und p-Phenylendiamin, 2,4-Diethyl-6-methyl-1,3-phenylendiamin, Bis(4-aminophenyl)-methan, Bis(4-aminophenyl)keton, Bis(4-aminophenyl)ether, Bis(4-aminophenyl)sulfid, Bis(3-aminophenyl)- und Bis(4-aminophenyl)sulfon, 4,4'-Diamino-3-ethyldiphenylmethan und Bis(4-amino-3-ethylphenyl)-methan.

Der Katalysator wird vorzugsweise in einem inerten Lösungsmittel, wie z.B. 2-Methoxyethanol, Isodecanol, Ethylenglykol, Diethylenglykol, N-Methylpyrrolidon, γ-butyrolacton, Benzylalkohol, Dibutylphthalat, Butan-1,4-diol, Ethyl-methylketon, Benzol oder Toluol, gelöst, dem Reaktionsgemisch beigegeben. Die Reaktion wird üblicherweise in einem inerten Lösungsmittel, wie z.B. in einem oder mehreren der oben genannten, bei erhöhter Temperatur, insbesondere bei 50 bis 100°C, durchgeführt. Das Epichlorhydrin und der Katalysator können je nach Wunsch auf einmal oder portionenweise beigegeben werden. Nachdem die Reaktion zwischen dem Amin und dem Epichlorhydrin beendet ist, üblicherweise nach etwa 1 bis 5 Stunden, wird auf konventionelle Art die Dehydrochlorierung durchgeführt, meistens durch Zugabe von Natrium- oder Kaliumhydroxid, gegebenenfalls zusammen mit einem quaternären Ammoniumhalogenid, wie z.B. Benzyltrimethylammoniumchlorid, als Katalysator. Nach Erhitzen bei 50-100°C während 2-10 Stunden, wird das Reaktionsgemisch mit Wasser gewaschen, und die organische Phase ergibt nach Entfernung der wässrigen Phase das gewünschte N-Glycidylamin. Dieses kann in rohem Zustand oder nach Reinigung gemäss üblichen Verfahren verwendet werden.

Die nach dem vorliegenden Verfahren erhaltenen N-Glycidylgruppen-enthaltenden Epoxidharze können auf übliche Weise gehärtet werden. Die vorliegende Erfindung umfasst auch Produkte, wie z.B. Giesskörper oder faserverstärkte Verbundwerkstoffe, die einen durch Härtung des nach dem erfindungsgemässen Verfahren erhaltenen Epoxidharzes hergestellten Stoff enthalten. Geeignete Härtungsmittel für N-Glycidylgruppen-enthaltende Epoxidharze sind wohl bekannt: sie umfassen z.B. Dicyandiamid, aromatische Amine, wie Bis(3-aminophenyl)- und Bis(4-aminophenyl)sulfon und Bis(4-aminophenyl)methan (meistens zusammen mit einem Härtungsbeschleuniger, wie z.B. einem $BF_3$-Amin-Komplex), sowie Anhydride von Polycarbonsäuren, wie Cyclohexan-1,2-dicarbonsäureanhydrid, Methylbicyclo[2.2.1]hept-5-en-2,3-dicarbonsäureanhydrid, Pyromellitsäuredianyhdrid, und Benzophenontetracarbonsäuredianhydrid.

Die folgenden Beispiele erläutern die Erfindung näher. Alle Teile und Prozente beziehen sich auf das Gewicht.

*Beispiel 1*

Bis(4-aminophenyl)methan (100 g), Toluol (100 ml) und 50%iges Magnesiumperchlorat in 2-Methoxyethanol (1 g) werden gerührt und auf 60°C erhitzt. Epichlorhydrin (203,5 g) wird portionenweise während zwei Stunden beigegeben, und die Temperatur wird zwischen 60 und 90°C gehalten. Nachdem das ganze Epichlorhydrin beigegeben ist, wird das Reaktionsgemisch noch eine Stunde auf 80°C gehalten.

Das Reaktionsgemisch wird auf 70°C abgekühlt und wird mit 6 g 50%igem wässrigem Benzyltrimethylammoniumchlorid behandelt. Flockenartiges Natriumhydroxid (89 g) wird portionenweise während 2 Stunden beigegeben, und das Reaktionsgemisch wird während 3 Stunden auf 75°C erhitzt. 300 ml Wasser und 250 ml Toluol werden unter kräftigem Rühren beigegeben, und das Reaktionsgemisch wird filtriert. Die wässrige Phase wird entfernt, und die organische Phase wird mit 250 ml einer Lösung aus 1310 Teilen Wasser, 450 Teilen Natriumchlorid und 9 Teilen Essigsäure gewaschen. Die organische Phase wird dann im Vakuum auf einem Rotationsverdampfer eingedampft. Das so erhaltene Produkt hat einen Epoxidgehalt von 8.62 Äquivalenten/kg (91% des theoretisch möglichen Wertes) und eine Viskosität bei 40°C von 46,5 Pa s.

Wenn das Experiment ohne das Magnesiumsalz als Katalysator wiederholt wird, erhält man ein Produkt mit einem Epoxidgehalt von 8,37 Äquivalenten/kg (88,3% der Theorie) und einer Viskosität bei 40°C von 70,7 Pa s.

*Beispiele 2-7*

Beispiel 1 wird wiederholt, indem man das Magnesiumperchlorat durch andere Salze in 2-Methoxyethanol ersetzt. Der Epoxidgehalt und die Viskosität der Produkte sind in der Tabelle angegeben.

| Beispiel | verwendeter Katalysator | | Produkt | |
|---|---|---|---|---|
| | Salz | Menge (g) | Epoxid- (Äquiv./kg) | Viskosität bei 40°C (Pa s) |
| 2 | Zinkperchlorat | 2 g | 8,63 | 45,6 |
| 3 | Calciumperchlorat | 2 g | 8.54 | 19.6 |
| 4 | Lanthannitrat | 1 g | 9.03 | 16,1 |
| 5 | Ytterbiumnitrat-Pentahydrat | 2 g | 8.96 | 26.2 |
| 6 | Uranylnitrat-Hexahydrat | 3 g | 8,61 | 44,8 |
| 7 | Nickelperchlorat | 2 g | 8,26 | 47,2 |

In den Beispielen 2, 3 und 5-7 wird das Salz in zwei gleichen Portionen am Anfang der Reaktion und nach Beendigung der Epichlorhydrinzugabe beigegeben.

*Beispiel 8*

2,4-Diethyl-6-methyl-1,3-phenylendiamin (100 g) wird auf 65°C erhitzt, und 50%iges Magnesiumperchlorat in Methoxyethanol (1 g) wird beigegeben. Während 2 Stunden wird Epichlorhydrin (207,9 g) portionenweise zugegeben, und das Reaktionsgemisch wird unterhalb 80°C gehalten. Nach beendeter Zugabe von Epichlorhydrin wird eine weitere Portion 50%iges Magnesiumperchlorat (1 g) beigegeben, und das Reaktionsgemisch wird während 8 Stunden bei 80°C erhitzt. Isopropanol (106 ml) wird beigegeben, und die Temperatur des Reaktionsgemisches wird auf 65°C eingestellt. Während zwei Stunden wird portionenweise 50%iges wässriges Natriumhydroxid (187,2g) beigegeben, und das Reaktionsgemisch wird während zwei weiteren Stunden bei 65°C gehalten. Nach Zugabe von Wasser (150 ml) und Ethyl-methylketon (250 ml) wird die wässrige Phase entfernt. Die organische Phase wird mit 250 ml der in Beispiel 1 beschriebenen Natriumchloridlösung gewaschen, in Vakuum am Rotationsverdampfer eingeengt und in 250 ml Ethylmethylketon aufgenommen. Filtration und Einengen ergeben ein Produkt mit einem Epoxidgehalt von 7,84 Äquivalenten/kg (78,8% der Theorie) und einer Viskosität bei 25°C von 21,5 Pa s.

Das Beispiel wird ohne Magnesiumperchlorat und mit einer Anfangsreaktion von 16 statt 8 Stunden wiederholt; das erhaltene Produkt hat einen Epoxidgehalt von 5,67 Äquivalenten/kg (57% der Theorie) und eine Viskosität bei 25°C von 581,9 Pa s.

*Beispiel 9*

Beispiel 8 wird wiederholt, indem statt Magnesiumperchlorat in zwei Portionen je 1,5 g einer 33%igen Lösung von Magnesiumnitrat in Methoxyethanol verwendet werden. Das Produkt hat einen Epoxidgehalt von 7,77 Äquivalenten/kg (78,1% der Theorie) und eine Viskosität bei 25°C von 27,5 Pa s.

*Beispiel 10*

Beispiel 1 wird wiederholt, indem das dort verwendete Amin durch ein flüssiges Gemisch von Bis(4-aminophenyl)methan, 4,4'-Diamino-3-ethyldiphenylmethan und Bis(4-amino-3-ethylphenyl)methan und das Magnesiumperchlorat durch Lanthannitrat ersetzt werden. Nachdem das ganze Epichlorhydrin beigegeben ist, wird das Reaktionsgemisch während 3 Stunden bei 80°C erhitzt. Das Produkt hat einen Epoxidgehalt von 8,23 Äquivalenten/kg (96,2% der Theorie) und eine Viskosität bei 25°C von 17,3 Pa s.

Wenn das Beispiel ohne Lanthannitrat als Katalysator und mit einer Anfangsreaktionszeit bei 80°C von 12 Stunden statt 3 Stunden wiederholt wird, enthält man ein Produkt mit einem Epoxidgehalt von 7,64 Äquivalenten/kg (89% der Theorie) und einer Viskosität bei 25°C von 50,3 Pa s.

*Beispiel 11*

Bis(4-aminophenyl)methan (100 g), Toluol (150 g) und 50%iges Mangannitrat in 2-Methoxyethanol (1 g) werden auf 60°C erhitzt und unter Vakuum (18665 Pa = 140 mm Hg) gestellt. Epichlorhydrin (203,5 g) wird portionenweise während 1 Stunde beigegeben. Am Ende der Zugabe wird das Vakuum aufgehoben, und die Temperatur wird auf 80°C erhöht. Es wird noch 1 g der Katalysatorlösung beigegeben, und das Reaktionsgemisch wird während 5 Stunden bei 80°C gehalten. 1,5 g 50%iges wässriges Benzyltrimethylammoniumchlorid werden beigegeben, und die Temperatur wird auf 75°C eingestellt. Natriumhydroxid (97,1 g) wird in 10 gleichen Portionen in 10minütigen Intervallen beigegeben; das Reaktionsgemisch wird dann noch während 1 Stunde auf 75°C gehalten und schliesslich mit 310 ml Wasser gewaschen. Die wässrige Phase wird entfernt, und die organische Phase wird mit der im Beispiel 1 beschriebenen Natriumchloridlösung gewaschen und im Vakuum eingedampft. Man erhält ein Produkt mit einem Epoxidgehalt von 8,48 Äquivalenten/kg (89,6% der Theorie) und einer Viskosität bei 50°C von 8,6 Pa s.

Wenn das Experiment ohne Mangannitrat wiederholt wird, erhält man ein Produkt mit einem Epoxidgehalt von 7,98 Äquivalenten/kg (84,3% der Theorie) und einer Viskosität bei 50°C von 9,8 Pa s.

*Beispiel 12*

Anilin (100 g), Toluol (150 g) und 50%iges Lanthannitrat in 2-Methoxyethanol (1 g) werden auf 60°C erhitzt und unter Vakuum (18665 Pa = 140 mm Hg) gestellt. Epichlorhydrin (216,6 g) wird portionenweise während 1 Stunde beigegeben. Das Vakuum wird aufgehoben und die Temperatur wird auf 80°C erhöht. Es wird noch 1 g der Katalysatorlösung beigegeben, das Reaktionsgemisch wird während 4 Stunden auf 80°C gehalten, dann werden 1,5 g 50%iges wässriges Benzyltrimethylammoniumchlorid beigegeben, und die Temperatur wird auf 75°C eingestellt. Natriumhydroxid (103,2 g) wird in 10 gleichen Portionen in 10minütigen Intervallen zugegeben, das Reaktionsgemisch wird während 1 Stunde auf 75°C gehalten und dann mit 350 ml Wasser gewaschen. Die wässrige Phase wird entfernt, und die organische Phase wird mit der im Beispiel 1 beschriebenen Natriumchloridlösung (250 ml) gewaschen und im Vakuum eingedampft. Man erhält ein Produkt mit einem Epoxidgehalt von 9,19 Äquivalenten/kg (94,4% der Theorie) und einer Viskosität bei 25°C von 0,09 Pa s.

Die Wiederholung des Experiments ohne Lanthannitrat ergibt ein Produkt mit einem Epoxidgehalt von 3,17 Äquivalenten/kg (32,5% der Theorie) und einer Viskosität bei 25°C von 51,1 Pa s.

*Beispiel 13*

Bis(3-aminophenyl)sulfon (50 g), Toluol (50 g) und 50%iges Lanthannitrat in 2-Methoxyethanol (5g) werden auf 60°C erhitzt und unter Vakuum (18665 Pa = 140 mm Hg) gestellt. Epichlorhydrin (81,3 g) wird während einer Stunde portionenweise beigegeben, das Vakuum wird aufgehoben, und die Temperatur wird auf 80°C erhöht. Es werden weitere 5 g Katalysatorlösung zugegeben, und das Reaktionsgemisch wird während 6 Stunden bei 80°C gehalten. Die Temperatur wird auf 75°C eingestellt und das Reaktionsgemisch wird mit 50%igem wässrigem Benzyltrimethylammoniumchlorid (0,75 g) behandelt. Natriumhydroxid (38,7 g) wird in 10 gleichen Portionen in 10minütigen Intervallen beigegeben, das Reaktionsgemisch wird während einer Stunde bei 75°C gehalten und dann mit 150 ml Wasser gewaschen. Die wässrige Phase wird entfernt, und die organische Phase wird mit der im Beispiel 1 beschriebenen Natriumchloridlösung (125 ml) gewaschen und im Vakuum eingedampft. Man erhält ein Produkt mit einem Epoxidgehalt von 7,40 Äquivalenten/kg (87,4% der Theorie) und einer Viskosität bei 50°C von 456,1 Pa s.

Wenn das Experiment ohne die Katalysatorlösung wiederholt wird, wird nach 6 Stunden bei 80°C eine ganz geringe Umsetzung festgestellt.

*Beispiel 14*

Ein Gemisch aus 1,3,3-Trimethyl-1-(4-aminophenyl)-5- und -6-aminoindanen (100 g), Toluol (150 g) und 50%igem Lanthannitrat in 2-Methoxyethanol (1 g) wird auf 60°C erhitzt und unter Vakuum (18665 Pa = 140 mm Hg) gestellt. Epichlorhydrin (151,5 g) wird während 1 Stunde portionenweise beigegeben, das Vakuum wird aufgehoben, und die Temperatur wird auf 80°C erhöht. Weitere Katalysatorlösung (1 g) wird zugegeben, und das Reaktionsgemisch wird während 5 Stunden auf 80°C gehalten. Die Temperatur wird dann auf 75°C eingestellt, und das Reaktionsgemisch wird mit 50%iger wässriger Benzyltrimethylammoniumchloridlösung (1,5 g) behandelt. Natriumhydroxid (72,3 g) wird in 10 gleichen Portionen in 10minütigen Intervallen beigegeben, das Reaktionsgemisch wird während einer Stunde auf 75°C gehalten und dann mit 250 ml Wasser gewaschen. Die wässrige Phase wird entfernt, und die organische Phase wird mit der im Beispiel 1 beschriebenen Natriumchloridlösung (250 ml) gewaschen und im Vakuum eingedampft. Man erhält ein Produkt mit einem Epoxidgehalt von 7,87 Äquivalenten/kg (96,6% der Theorie) und einer Viskosität bei 50°C von 121,7 Pa s.

Bei Wiederholung des Experiments ohne Lanthannitrat erhält man ein Produkt mit einem Epoxidgehalt von 6,06 Äquivalenten pro kg (74,7% der Theorie) und einer Viskosität bei 50°C von 220 Pa s.

*Beispiel 15*

Bis(4-aminophenyl)methan (100 g), Toluol (100 g) und 50%iges Magnesiumtrifluormethansulfonat in 2-Methoxyethanol (1 g) werden gemischt und auf 60°C erhitzt. Epichlorhydrin (203,5 g) wird portionenweise während zwei Stunden beigegeben und die Temperatur wird zwischen 60 und 80°C gehalten. Nach beendeter Zugabe wird das Reaktionsgemisch noch eine Stunde bei 80°C gehalten, auf 75°C gekühlt, und 6 g einer 50%iges wässrigen Benzyltrimethylammoniumchloridlösung werden beigegeben. Das Reaktiongemisch wird mit Natriumhydroxid (89 g), das während 2 Stunden portionsweise beigegeben wird, behandelt und dann noch weitere 3 Stunden auf 75°C gehalten. 300 ml Wasser und 250 ml Toluol werden mit kräftigem Rühren beigegeben, das Reaktionsgemisch wird filtriert, und die wässrige Phase wird entfernt. Die organische Phase wird mit einer Lösung (250 ml) aus 1310 Teilen Wasser, 450 Teilen Natriumchlorid und 9 Teilen Essigsäure gewaschen und im Vakuum auf einem Rotationsverdampfer eingedampft. Man erhält ein Produkt mit einem Epoxidgehalt von 8,87 Äquivalenten/kg (93,6% der Theorie) und einer Viskosität bei 40°C von 16,2 Pa s.

Bei Wiederholung der Reaktion ohne Katalysator erhält man ein Produkt mit einem Epoxidgehalt von 8,37 Äquivalenten pro kg (88,3% der Theorie) und einer Viskosität bei 40°C von 70,7 Pa s.

*Beispiele 16-19*

Beispiel 15 wird wiederholt, indem man 1 g des Magnesiumtrifluormethansulfonats durch 2 g der unten angegebenen Salze als 50%ige Lösung in Methoxyethanol ersetzt. Der Katalysator wird jeweils in zwei gleichen Portionen vor und nach der Zugabe von Epichlorhydrin beigegeben. Die eingesetzten Katalysatoren und die Eigenschaften der erhaltenen Produkte sind in der folgenden Tabelle angegeben.

| Beispiel | Verwendetes Salz | Produkt | |
|---|---|---|---|
| | | Epoxidgehalt (Äquival./kg) | Viskosität (Pa s) bei 40°C |
| 16 | Magnesiumtrifluoracetat | 8,86 | 19.6 |
| 17 | Magnesiumtrifluoracetat | 8,73 | 48,7 |
| 18 | Kobalttrifluormethanesulfonat | 9,12 | 18,5 |
| 19 | Nickeltrifluoracetat | 8,40 | 54,3 |

*Beispiel 20*

2,4-Diethyl-6-methyl-1,3-phenylendiamin (100 g) wird auf 65°C erhitzt, und 50%iges Magnesiumtrifluormethansulfonat in Methoxyethanol (1 g) wird beigegeben. Epichlorhydrin (207,9) wird während 2 Stunden portionenweise zugegeben, und die Temperatur wird unterhalb 80°C gehalten. Nach der Zugabe des Epichlorhydrins wird zusätzlich noch 1 g der 50%igen Natriumtrifluormethansulfonatlösung beigegeben, und das Gemisch wird während 4 Stunden auf 80°C gehalten. Isopropanol (106 ml) wird beigegeben, und die Temperatur des Reaktionsgemisches wird auf 65°C eingestellt. 50%iges wässriges Natriumhydroxid (187,2 g) wird während 2 Stunden portionenweise beigegeben, und das Reaktionsgemisch wird während weiteren 2 Stunden bei 65°C gehalten. Anschliessend werden Wasser (150 ml) und Ethylmethylketon (250 ml) zugegeben, und die wässrige Phase wird entfernt. Die organische Phase wird mit der im Beispiel 1 beschriebenen Natriumchloridlösung (250 ml), auf einem Rotationsverdampfer im Vakuum eingedampft und in Ethyl-methylketon (250 ml) wieder gelöst. Nach der Filtration und Entfernung des Lösungsmittels wird ein Produkt mit einem Epoxidgehalt von 7,90 Äquivalenten/kg (79,4% der Theorie) und einer Viskosität bei 25°C von 20,0 Pa s erhalten.

Nach Wiederholung des Beispiels ohne Magnesiumsalz und mit einer Anfangsreaktionszeit von 16 statt 4 Stunden, erhält man ein Produkt mit einem Epoxidgehalt von 5,67 Äquivalenten/kg (57% der Theorie) und einer Viskosität von 581,9 Pa s bei 25°C.

*Beispiel 21*

Beispiel 20 wird unter Verwendung von Magnesiumtrifluoracetat statt Trifluormethansulfonat wiederholt. Das Produkt hat einen Epoxidgehalt von 8,08 Äquivalenten/kg (81,2% der Theorie) und eine Viskosität bei 25°C von 31,5 Pa s.

*Beispiel 22*

Ein flüssiges Gemisch aus Bis(4-aminophenyl)methan, 4,4'-Diamino-3-ethyldiphenylmethan und Bis-

(4-amino-3-ethylphenyl)methan (100 g), Toluol (100 ml) und 50%iges Magnesiumtrifluormethansulfonat in Methoxyethanol (1 g) wird gerührt und auf 60°C erhitzt. Epichlorhydrin (165,2 g) wird portionenweise während 2 Stunden beigegeben, und die Reaktionstemperatur wird unterhalb 80°C gehalten. Nach Beendigung der Zugabe wird das Gemisch während weiterer 4 Stunden bei 80°C erhitzt, auf 75°C gekühlt, 6 g 50%iges wässriges Benzyltrimethylammoniumchlorid werden beigegeben, gefolgt von portionenweiser Zugabe von 72,3 g Natriumhydroxid während 2 Stunden. Das Gemisch wird während 3 Stunden bei 75°C gehalten und dann mit Wasser (250 ml) Toluol (250 ml) gewaschen. Die organische Phase wird von der wässrigen Phase getrennt, mit der in Beispiel 1 beschriebenen Natriumchloridlösung (250 ml) gewaschen und eingedampft. Der Rest wird in Toluol (250 ml) wieder gelöst, filtriert und eingedampft. Man erhält ein Produkt mit einem Epoxidgehalt von 8,07 Äquivalenten/kg (94,4% der Theorie) und einer Viskosität bei 25°C von 33,4 Pa s.

Nach Wiederholung des Beispiels ohne das Magnesiumsalz und mit einer Anfangsreaktionszeit von 12 statt 4 Stunden bei 80°C erhält man ein Produkt mit einem Epoxidgehalt von 7,64 Äquivalenten/kg (89% der Theorie) und einer Viskosität bei 25°C von 50,3 Pa s.

*Beispiel 23*

Bis(4-aminophenyl)sulfon (100 g), Toluol (100 ml) und 50%iges Magnesiumtrifluormethansulfonat in Methoxyethanol (1 g) werden gerührt und auf 65°C erhitzt. Epichlorhydrin (162,5 g) wird während 2 Stunden portionenweise beigegeben, und die Temperatur des Gemisches wird unterhalb von 80°C gehalten. Weitere 1 g der Magnesiumlösung werden beigegeben, und das Gemisch wird während 13 Stunden bei 80°C gehalten. Nach dieser Zeit beträgt der Epoxidgehalt des Gemisches 0,64 Äquivalente/kg.

Das Toluol wird auf einem Rotationsverdampfer eingedampft, und der Rest wird in Isopropanol (100 ml) aufgeschlämmt. 50%iges wässriges Natriumhydroxid (160 g) wird während 1 Stunde bei 65°C beigegeben, und das Gemisch wird eine weitere Stunde bei 65°C gehalten. Wasser (300 ml) und Ethylmethylketon (300 ml) werden beigegeben, die wässrige Phase wird entfernt, und die organische Phase wird mit der im Beispiel 1 beschriebenen Natriumchloridlösung (250 ml) gewaschen und eingedampft. Man erhält ein festes Produkt mit einem Epoxidgehalt von 6,46 Äquivalenten/kg (76,2% der Theorie).

Nach Wiederholung des Experiments ohne das Magnesiumsalz und mit einer Anfangsreaktionszeit von 18 Stunden bei 80°C wird nur ein kleiner Abfall des Epoxidgehalts (von 5,04 auf 4,40 Äquivalente pro kg) festgestellt, was zeigt, dass die erste Reaktionsstufe nicht stattfindet.

*Beispiel 24*

Beispiel 11 wird wiederholt, indem man die Mangannitratlösung durch eine 50%ige lösung von Magnesiumtrichloracetat in Isodecanol ersetzt. Das Produkt hat einen Epoxidgehalt von 9,19 Äquivalenten/kg (97,1% der Theorie) und eine Viskosität bei 50°C von 4,1 Pa s.

*Beispiel 25*

Beispiel 12 wird wiederholt, indem man Magnesiumtrifluormethansulfonat statt Lanthannitrat verwendet. Das Produkt hat einen Epoxidgehalt von 9,18 Äquivalenten/kg (94,3% der Theorie) und eine Viskosität bei 25°C von 0,06 Pa s.

*Beispiel 26*

Beispiel 14 wird wiederholt, indem man Lanthannitrat durch Magnesiumtrifluormethansulfonat ersetzt. Das Produkt hat einen Epoxidgehalt von 7,45 Äquivalenten/kg (91,4% der Theorie) und eine Viskosität bei 50°C von 84,3 Pa s.

*Beispiele 27-29*

Beispiel 24 wird wiederholt, indem man die Magnesiumtrichloracetatlösung durch 50%ige Lösung der unten angegebenen Salze in 2-Methoxyethanol ersetzt. Die Epoxidgehalte und Viskositäten sind unten angegeben:

| Beispiel | Katalysator | Produkt | |
|---|---|---|---|
| | | Epoxidgehalt (Äquival./kg) | Viskosität (Pa s) bei 50°C |
| 27 | Vanadyltrifluoracetat | 8,72 | 7,2 |
| 28 | Magnesiumtribromacetat | 8,51 | 7,9 |
| 29 | Magnesium-2,2-dichlorpropionat | 8,56 | 5,8 |

**Patentansprüche**

1. Verfahren zur Herstellung von aromatischen N-Glycidylaminen, worin ein Amin, das mindestens ein aromatisches Aminwasserstoffatom enthält, mit mindestens 0,7 Äquivalenten Epichlorhydrin pro Aminwasserstoffäquivalent des aromatischen Amins, in Anwesenheit eines zwei- oder mehrwertigen Metallsalzes (a) der Salpeter- oder Perchlorsäure oder (b) einer Carbon- oder Sulfonsäure, die durch Fluor, Chlor oder Brom am α-Kohlenstoff zur Carboxyl- oder Sulfonsäuregruppe substituiert ist, erhitzt wird, und anschliessend das Produkt dehydrochloriert wird.

2. Verfahren nach Anspruch 1, worin das als Katalysator verwendete Salz ein Nitrat oder Perchlorat eines Metalls der Gruppe IIa, IIb, IIIb, VIIb oder VIII des periodischen Systems, wie im «Handbook of Chemistry», Lange, 12. Auflage, McGraw-Hill veröffentlicht, oder ein Fluor-, Chlor- oder Brom-substituiertes Carboxylat oder Sulfonat eines Metalls der Gruppe IIa oder eines Übergangsmetalls ist.

3. Verfahren nach Anspruch 2, worin das verwendete Salz ein Nitrat oder Perchlorat des Magnesiums, Calciums, Zinks, Mangans, Nickels, Lanthans, Vanadiums (als Vanadyl), Ytterbiums oder Uraniums (als Uranyl), oder ein Fluor-, Chlor- oder Brom-substituiertes Carboxylat oder Sulfonat des Eisens, Zinks, Cadmiums, Lanthans, Magnesiums, Vanadiums (als Vanadyl), Mangans, Kobalts oder Nickels ist.

4. Verfahren nach Anspruch 3, worin das verwendete Salz Magnesiumperchlorat, Calciumperchlorat, Zinkperchlorat, Nickelperchlorat, Magnesiumnitrat, Mangannitrat, Lanthannitrat, Ytterbiumnitrat oder Uranylnitrat ist.

5. Verfahren nach Anspruch 3, worin das Anion des Salzes von einer höchstens 4 Kohlenstoffatome enthaltenden Carbon- oder Sulfonsäure abgeleitet ist.

6. Verfahren nach Anspruch 5, worin das Anion des Salzes von Trifluoressigsäure, Trifluormethansulfonsäure, Trichloressigsäure, 2,2-Dichlorpropionsäure oder Tribromessigsäure abgeleitet ist.

7. Verfahren nach Anspruch 6, worin das Salz Magnesiumtrifluoracetat, Mangantrifluoracetat, Nickeltrifluoracetat, Vanadyltrifluoracetat, Magnesiumtrifluormethansulfonat, Kobalttrifluormethansulfonat, Magnesiumtrichloracetat, Magnesium-2,2-dichlorpropionat oder Magnesiumtribromacetat ist.

8. Verfahren nach einem der vorgehenden Ansprüche, worin die verwendete Salzmenge 0,1 bis 10 Gewichtsteile pro 100 Gewichtsteile des aromatischen Amins beträgt.

9. Verfahren nach einem der vorangehenden Ansprüche, worin der Katalysator dem Reaktionsgemisch in einem inerten Lösungsmittel gelöst beigegeben wird.

10. Verfahren nach einem der vorgehenden Ansprüche, worin das aromatische Amin eine oder zwei primäre Aminogruppen hat.

11. Verfahren nach Anspruch 10, worin das aromatische Amin ein Anilin, ein Aminophenylindan oder ein Amin der Formel I oder II

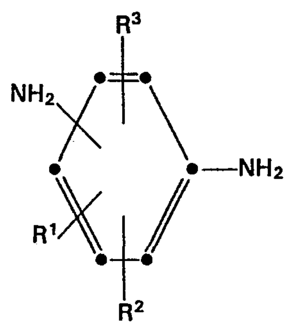

I

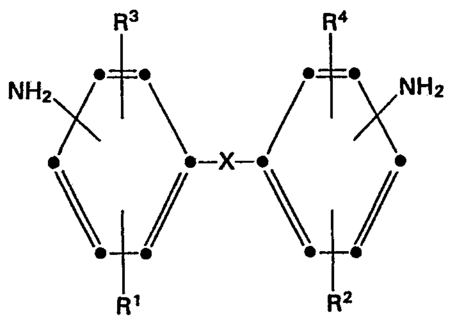

II

ist, worin

$R^1$, $R^2$, $R^3$ und $R^4$ gleich oder verschieden sind und Alkylgruppen mit 1 bis 4 Kohlenstoffatomen oder Wasserstoffatome darstellen, und X eine direkte Bindung, eine Alkylengruppe mit 1 bis 4 Kohlenstoffatomen, ein Sauerstoffatom, ein Schwefelatom oder eine Carbonyl- oder Sulfonylgruppe darstellt.

12. Verfahren nach Anspruch 11, worin das aromatische Amin Anilin, 1,3,3-Trimethyl-1-(4-aminophenyl)-5-aminoindan, 1,3,3-Trimethyl-1-(4-aminophenyl)-6-aminoindan, o-, m- oder p-Phenylendiamin, 2,4-Diethyl-6-methyl-1,3-phenylendiamin, Bis(4-aminophenyl)methan, Bis(4-aminophenyl)keton, Bis(4-aminophenol)ether, Bis(4-aminophenyl)-sulfid, Bis(3-aminophenyl)- oder Bis(4-aminophenyl)sulfon, 4,4'-Diamino-3-ethyldiphenylmethan oder Bis(4-amino-3-ethylphenyl)methan ist.

13. Verfahren nach einem der vorgehenden Ansprüche , das bei Temperaturen von 50 bis 100°C durchgeführt wird.

## Claims

1. A process for the preparation of aromatic N-glycidylamines which comprises heating an amine having at least one aromatic amino hydrogen atom with at least 0.7 equivalent, per amino hydrogen equivalent of the amine, of epichlorohydrin, in the presence of a divalent or polyvalent metal salt of (a) nitric or perchloric acid, or (b) a carboxylic or sulfonic acid substituted by fluorine, chlorine or bromine on a carbon atom alpha to the carboxyl or sulfo group, and dehydrochlorinating the product.

2. A process according to claim 1, in which the salt used as catalyst is a nitrate or perchlorate of a metal of group IIa, IIb, IIIb, VIIb or VIII of the Periodic Table, as published in the «Handbook of Chemistry», Lange, 12th Edition, McGraw-Hill, or a fluorine-, chlorine- or bromine-substituted carboxylate or sulfonate of a metal of group IIa or a transition metal.

3. A process according to claim 2, in with the salt used is a nitrate or perchlorate of magnesium, calcium, zinc, manganese, nickel, lanthanum, vanadium (as vanadyl), ytterbium, or uranium (as uranyl) or a fluorine-, chlorine- or bromine-substituted carboxylate or sulfonate of iron, zinc, cadmium, lanthanum, magnesium, vanadium (as vanadyl), manganese cobalt or nickel.

4. A process according to claim 3, in which the salt used is magnesium perchlorate, calcium perchlorate, zinc perchlorate, nickel perchlorate, magnesium nitrate, manganese nitrate, lanthanum nitrate, ytterbium nitrate, or uranyl nitrate.

5. A process according to claim 3, in which the anion of the salt is derived from a substituted carboxylic or sulfonic acid having at most 4 carbon atoms.

6. A process according to claim 5, in which the anion of the salt is derived from trifluoroacetic acid, trifluoromethansulfonic acid, trichloroacetic acid, 2,2-dichloropropionic acid or tribromoacetic acid.

7. A process according to claim 6, in which the salt is magnesium trifluoroacetate, manganese trifluoroacetate, nickel trifluoroacetate, vanadyl trifluoroacetate, magnesium trifluoromethanesulfonate, cobalt trifluoromethanesulfonate, magnesium trichloroacetate, magnesium 2,2-dichloropropionate, or magnesium tribromoacetate.

8. A process according to any of the preceding claims, in which the amount of the salt used is from 0.1 to 10 parts by weight per 100 parts by weight of the aromatic amine.

9. A process according to any of the preceding claims, in which the catalyst is incorporated into the reaction mixture dissolved in an inert solvent.

10. A process according to any of the preceding claims, in which the aromatic amine has one or two primary amino groups.

11. A process according to claim 10, in which the aromatic amine is an aniline, an aminophenylindane or an amine of the formula I or II:

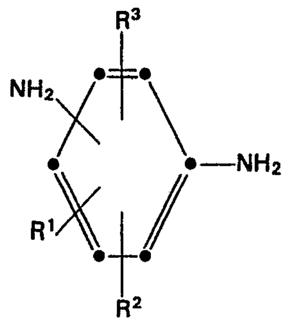

I

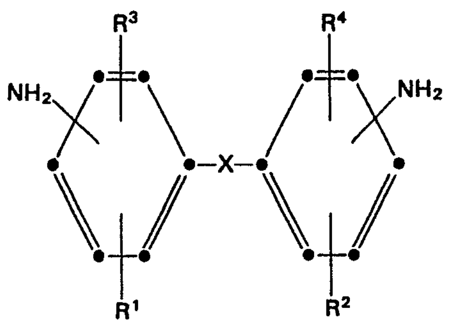

II

where

$R^1$, $R^2$, $R^3$ and $R^4$ are the same or different and represent alkyl groups of from 1 to 4 carbon atoms, or hydrogen atoms, and

X represents a direct bond, an alkylene group of from 1 to 4 carbon atoms, an oxygen atom, a sulphur atom or a carbonyl or sulfonyl group.

12. A process according to claim 11, in which the aromatic amine is aniline, 1,3,3-trimethyl-1-(4-aminophenyl)-5-aminoindane, 1,3,3-trimethyl-1-(4-aminophenyl)-6-aminoindane, o-, m-, or p-phenylenediamine, 2,4-diethyl-6-methyl-1,3-phenylenediamine, bis(4-aminophenyl) methane, bis(4-aminophenyl) ketone, bis(4-aminophenyl) ether, bis(4-aminophenyl) sulfide, bis(3-aminophenyl) or bis(4-aminophenyl) sulfone, 4,4' diamino-3-ethyldiphenylmethane or bis(4-amino-3-ethylphenyl)methane.

13. A process according to any of the preceding claims, which is effected at temperatures of 50 to 100°C.

**Revendications**

1. Procédé de préparation de N-glycidylamines aromatiques, procédé caractérisé en ce qu'on chauffe une amine ayant au moins un atome d'hydrogène aminé aromatique avec au moins 0,7 équivalent d'épichlorhydrine par équivalent d'hydrogène aminé de l'amine aromatique, en présence d'un sel de métal de di- ou poly-valent (a) de l'acide nitrique ou de l'acide perchlorique ou (b) d'un acide carboxylique ou sulfonique portant un atome de fluor, de chlore ou de brome sur l'atome de carbone en position α relativement au radical carboxy ou sulfo, puis on déshydrochlore le produit formé.

2. Procédé selon la revendication 1 dans lequel le sel utilisé comme catalyseur est un nitrate ou un perchlorate d'un métal appartenant à l'un des groupes IIa, IIb, IIIa, VIIb et VIII de la classification périodique des éléments, telle qu'elle est publiée dans «Handbook of Chemistry», Lange, 12ème, éd. McGraw-Hill, ou in carboxylate ou sulfonate fluoré, chloré ou bromé d'un métal du groupe IIa ou d'un métal de transition.

3. Procédé selon la revendication 2 dans lequel le sel utilisé est un nitrate ou un perchlorate du magnésium, du calcium, du zinc, du manganèse, du nickel, du lanthane, du vanadium (à l'état de vanadyle), de l'ytterbium ou de l'uranium (à l'état d'uranyle), ou un carboxylate ou sulfonate fluoré, chloré ou bromé du fer, du zinc, du cadmium, du lanthane, du magnésium, du vanadium (à l'état de vanadyle), du magnésium, du cobalt ou du nickel.

4. Procédé selon la revendication 3 dans lequel le sel utilisé est le perchlorate de magnésium, le perchlorate de calcium, le perchlorate de zinc, le perchlorate de nickel, le nitrate de magnésium, le nitrate de manganèse, le nitrate de lanthane, le nitrate d'ytterbium ou le nitrate d'uranyle.

5. Procédé selon la revendication 3 dans lequel l'anion du sel est celui d'un acide carboxylique ou sulfonique contenant au maximum 4 atomes de carbone.

6. Procédé selon la revendication 5 dans lequel l'anion du sel est celui de l'acide trifluoracétique, de l'acide trifluorométhanesulfonique, de l'acide trichloracétique, de l'acide dichloro-2,2 propionique ou de l'acide tribromacétique.

7. Procédé selon la revendication 6 dans lequel le sel est le trifluoracétate de magnésium, le trifluoracétate de manganèse, le trifluoracétate de nickel, le trifluoracétate de vanadyle, le trifluorométhanesulfonate de magnésium, le trifluorométhanesulfonate de cobalt, le trichloroacétate de magnésium, le dichloro-2,2-propionate de magnésium ou le tribromoacétate de magnésium.

8. Procédé selon l'une quelconque des revendications précédentes dans lequel la quantité de sel utilisée est comprise entre 0,1 et 10 parties en poids pour 100 parties en pois de l'amine aromatique.

9. Procédé selon l'une quelconque des revendications précédentes dans lequel le catalyseur est ajouté sous la forme d'une solution dans un solvant inerte au mélange réactionnel.

10. Procédé selon l'une quelconque des revendications précédentes dans lequel l'amine aromatique contient 1 ou 2 radicaux amino primaires.

11. Procédé selon la revendication 10 dans lequel l'amine aromatique est une aniline, un aminophénylindane ou une amine répondant à l'une des formules I et II:

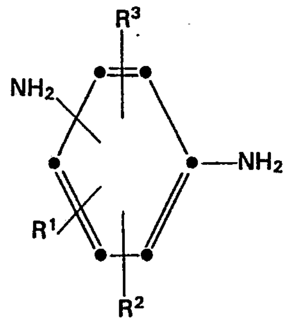

I

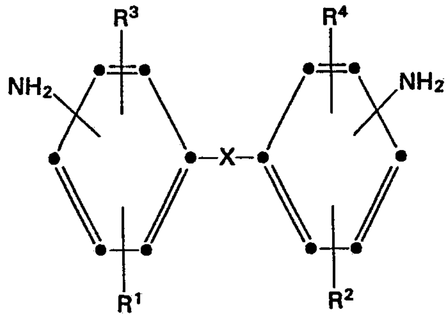

II

dans lesquelles $R^1$, $R^2$, $R^3$ et $R^4$ représentent chacun, indépendamment les uns des autres, un alkyle contenant de 1 à 4 atomes de carbone ou un atome d'hydrogène, et X représente une liaison directe, un alkylène contenant de 1 à 4 atomes de carbone, un atome d'oxygène, un atome de soufre ou un radical carbonyle ou sulfonyle.

12. Procédé selon la revendication 11 dans lequel l'amine aromatique est l'aniline, le triméthyl-1,3,3 (amino-4 phényl)-1 amino-5 indane, le triméthyl-1,3,3 (amino-4 phényl)-1 amino-6 indane, la o-, s- ou p-phénylène-diamine, le diéthyl-2,4 méthyl-6 diamino-1,3 benzène, le bis-(amino-4 phényl)-méthane, la bis-(amino-4 phényl)-cétone, l'oxyde de bis-(amino-4 phényle), le sulfure de bis-(amino-4 phényle), la bis-(amino-3 phényl)- ou bis-(amino-4 phényl)-sulfone, le diamino-4,4' éthyl-3 diphénylméthyne ou le bis-(amino-4 éthyl-3 phényl)-méthane.

13. Procédé selon l'une quelconque des revendications précédentes qui est executé à des températures de 50 à 100°C.